# EUROPEAN PATENT SPECIFICATION

(11) **EP 1 896 844 B1**
(45) Date of publication and mention of the grant of the patent: **07.08.2013**
(21) Application number: 06780775.0
(22) Date of filing: 28.06.2006
(51) Int. Cl.: G01N 33/14, G01N 30/88

(54) **METHOD FOR PROCYANIDIN ANALYSIS**
PROCYANIDIN-ANALYSEVERFAHREN
PROCEDE D'ANALYSE DE PROCYANIDINE

(30) Priority: 30.06.2005 JP 2005191665
(43) Date of publication of application: 12.03.2008
(60) Divisional application: 11155795.5
(73) Proprietor: Suntory Holdings Limited, Kita-ku, Osaka-shi Osaka 530-8203 (JP)
(72) Inventor: FUKUI, Yuko, 5691123 (JP)
(74) Representative: Vossius & Partner
(86) International application number: PCT/JP2006/313348
(87) International publication number: WO 2007/004659

(56) References cited:
- PEREZ-ILZARBE F J ET AL: "LIQUID CHROMATOGRAPHIC DETERMINATION OF APPLE PULP PROCYANIDINS" JOURNAL OF LIQUID CHROMATOGRAPHY, NEW YORK, NY, US, vol. 15, no. 4, 1992, pages 637-646, XP008057047 ISSN: 0148-3919
- KARONEN M ET AL: "Analysis of procyanidins in pine bark with reversed-phase and normal-phase high-performance liquid chromatography-electrospray ionization mass spectrometry" ANALYTICA CHIMICA ACTA, ELSEVIER, AMSTERDAM, NL, vol. 522, no. 1, 20 September 2004 (2004-09-20), pages 105-112, XP004549839 ISSN: 0003-2670
- YANAGIDA A ET AL: "Fractionation of apple procyanidins by size-exclusion chromatography" JOURNAL OF CHROMATOGRAPHY A, ELSEVIER, AMSTERDAM, NL, vol. 855, no. 1, 3 September 1999 (1999-09-03), pages 181-190, XP004180025 ISSN: 0021-9673
- ESCARPA A ET AL: "Fast separation of (poly)phenolic compounds from apples and pears by high-performance liquid chromatography with diode-array detection" JOURNAL OF CHROMATOGRAPHY A, ELSEVIER, AMSTERDAM, NL, vol. 830, no. 2, 15 January 1999 (1999-01-15), pages 301-309, XP004153877 ISSN: 0021-9673
- LIN ET AL: "Survey of Catechins, Gallic Acid, and Methylxanthines in Green, Oolong, Pu-erh, and Black Teas" JOURNAL OF AGRICULTURAL AND FOOD CHEMISTRY, AMERICAN CHEMICAL SOCIETY. WASHINGTON, US, vol. 46, no. 9, 1998, pages 3635-3642, XP002991389 ISSN: 0021-8561

## Description

### FIELD OF THE INVENTION

The present invention relates to a method for quantifying procyanidin and flavan-3-ols contained in a tea product, foods and/or drinks.

### BACKGROUND OF THE INVENTION

In terms of efficacy, proanthocyanidin (OPC) is considered to be among the active ingredients related to "French Paradox" because OPC is also contained in wines (1995, Clin. Chim. Acta., 235, 207-219). OPC is known to have various actions and effects such as an antioxidative action, a peripheral circulation-improving action, a blood flow-improving effect and a liver function-improving effect (2004, Japan Food Science, 403, January issue, 40-45), as well as a platelet aggregation-inhibiting effect (JP 2003-527418 A).

Previously-known techniques for proanthocyanidin analysis include reversed-phase HPLC by using a high performance liquid chromatograph-mass spectrometer (LC-MS) (2003, Biosci. Biotechnol. Biochem., 67(5), 1140-1142) and normal phase HPLC by LC-MS using gradient elution (2003, J. Agric. Food Chem., 51, 7513-7521). Techniques for procyanidin analysis using a pretreatment column before effecting reversed-phase HPLC are also known (2004, Analyt. Chim. Acta, 522(1), 105-112; 1999, J. Chromatogr. A, 830(2), 301-309). However, in either of these analysis techniques, procyanidin in the form of a mixture with other ingredients from tea, food or supplements has been difficult to separate from such contaminant peaks; thus, these techniques cannot achieve accurate quantification. Moreover, there are a very large number of stereoisomers for proanthocyanidin due to the stereoisomerism of its constituents, flavan-3-ols, and compounds available as standards have therefore been limited. For this reason, quantitative analysis has been impossible, except for some known compounds.

### SUMMARY OF THE INVENTION

There is a strong demand for the development of a novel analysis method that enables accurate quantification of procyanidin (a generic name for a mixture of catechin n-mer; n ≧ 1) and flavan-3-ols in tea products, foods and/or drinks, which contain procyanidin and flavan-3-ols, without receiving any interference from contaminants. Thus, the present invention provides a novel method for quantifying procyanidin and flavan-3-ols contained in tea products, foods and/or drinks.

### BRIEF DESCRIPTION OF THE DRAWINGS

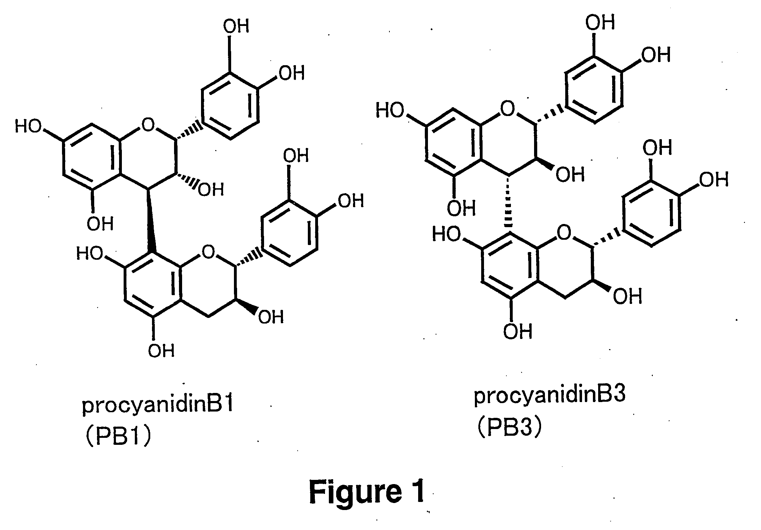

Figure 1 shows the structural formulae of procyanidin B1 and procyanidin B3.

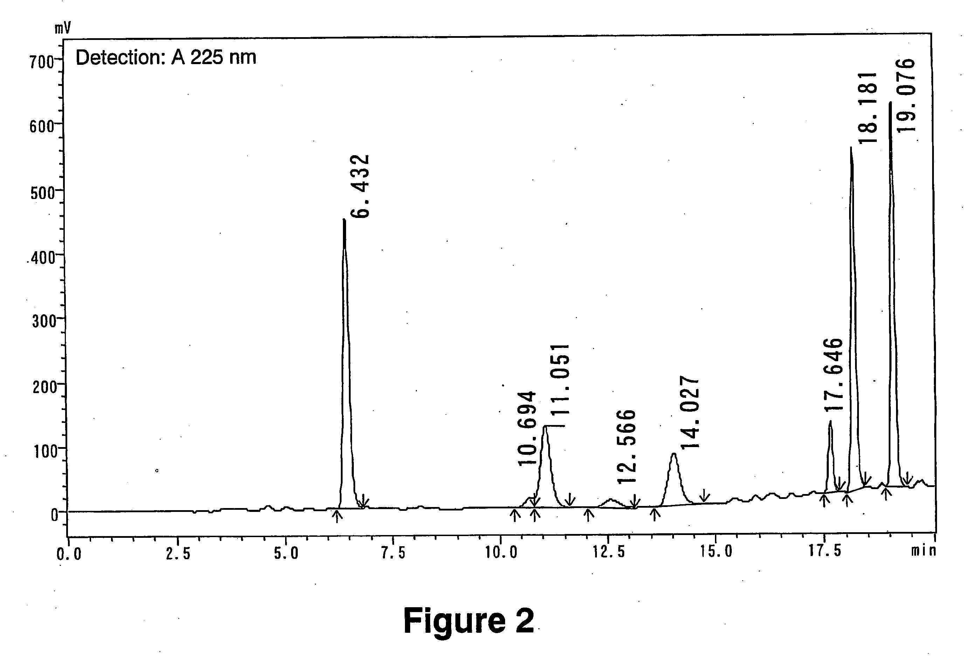

Figure 2 shows an HPLC chromatogram of the fraction eluted with 70% EtOH in the pretreatment step. Analysis conditions are as follows: HPLC system: Shimadzu LC-2010HT (Shimadzu Corporation, Japan); Column: capcellpak C-18 AQ (6 mm x 150 mm, Shiseido Co., Ltd., Japan); Mobile phase: Eluent A: 0.05% TFA/water, Eluent B: 0.05% TFA/90% CH₃CN/water; Flow rate: 1.2 ml/minute; Gradient: 10% B → 10% B (10 minutes) and 10% B → 35% B (10 minutes); Column temperature: 40°C; Detection: A225 nm. Under these conditions, procyanidin B1 was eluted at 10.7 minutes, procyanidin B3 at 12.6 minutes, catechin at 14.0 minutes, epicatechin at 17.6 minutes, gallocatechin at 6.4 minutes, epigallocatechin at 11.1 minutes, epigallocatechin-3-O-gallate at 18.2 minutes, and gallocatechin-3-O-gallate at 19.1 minutes, respectively.

### DETAILED DESCRIPTION OF THE INVENTION

As a result of various studies made to achieve the above object, the inventors of the present invention have found that procyanidin and flavan-3-ols contained in tea products and/or foods and drinks can be quantified without being affected by contaminants when (a) pretreating a test sample with a dextran-based or vinyl polymer-based resin to remove contaminants (e.g., caffeine) which cannot be separated using hydrophobic adsorbent resins or the like, and (b) effecting high performance liquid chromatography to separate and quantify the fractionated procyanidin and flavan-3-ols.

Thus, the present invention provides a method for quantifying procyanidin and flavan-3-ols contained in a tea product, foods and/or drinks according to claim 1.

### Substances to be quantified by the method of the present invention

The substance to be quantified by the method of the present invention may be any of procyanidin (a generic name for a mixture of catechin n-mer; n ≧ 1) and flavan-3-ols. Flavan-3-ols to be quantified by the method of the present invention include, but are not limited to, catechin, epicatechin, gallocatechin, epigallocatechin, catechin-3-O-gallate, epicatechin-3-O-gallate, gallocatechin-3-O-gallate and epigallocatechin-3-O-gallate. Procyanidin to be quantified by the method of the present invention include, but are not limited to, procyanidins B1, B2, B3, B4, B5, B6, B7 and B8. The substance to be quantified by the method of the present invention may preferably be procyanidin B1 (PB1) and/or procyanidin B3 (PB3), and more preferably PB1.

### Test samples

The test sample to be analyzed by the method of the present invention may be any sample expected to contain procyanidin and/or flavan-3-ols selected from the group consisting of a tea product, foods and/or drinks (e.g., grape seeds, tamarind, apple, bark, tea leaves, cocoa and/or treated products (e.g., extracts) thereof). A preferred test sample to be analyzed by the method of the present invention is a tea beverage containing procyanidin, and more preferably a tea beverage containing a pine bark extract.

### Pretreatment step

In the analysis of procyanidin and flavan-3-ols in a test sample using high performance liquid chromatography, contaminants which disturb the analysis include methylxanthines (e.g., caffeine, theobromine), phenylpropanoids (e.g., coffeic acid, coumaric acid, ferulic acid), flavones, flavonols, and glycosylated compounds thereof.

When a reversed-phase (e.g., C8 or C18) carrier is used in an attempt to separate procyanidin and flavan-3-ols from these contaminants, these substances are all adsorbed on the resin and hence eluted at the same time. For this reason, they are impossible to separate from each other or, even where possible, require a very long period of time for their analysis. After a series of studies about conditions which allow separation of procyanidin and flavan-3-ols from these contaminants, the inventors of the present invention have succeeded in separating procyanidin and flavan-3-ols from these contaminants on a gel filtration column with an eluent containing a polar organic solvent. Thus, in the method of the present invention, column separation enables the fractionation of procyanidin and flavan-3-ols from the above contaminants when it is used as a pretreatment.

Column resins available for use in the pretreatment include dextran-based resins (e.g., Sephadex LH-20 (Amersham Biosciences)) and hydrophilic vinyl polymer-based gel filtration resins (e.g., TOYOPEARL HW40 (Tosoh Corporation, Japan), TOYOPEARL HW-50 (Tosoh Corporation, Japan)). These resins enable the fractionation between contaminants and procyanidin/flavan-3-ols.

In the pretreatment step, contaminants are eluted with water and/or 0% to 40% ethanol, and then procyanidin and flavan-3-ols are collected with 60% or more ethanol. For example, after a test sample is charged onto a column, caffeine as a contaminant may be eluted with 0% to 20% ethanol, while phenylpropanoids, flavones and flavonols may be eluted with 20% to 40% ethanol, and finally followed by elution of procyanidin and flavan-3-ols with 60% to 80% ethanol to obtain the desired procyanidin and flavan-3-ols without containing any contaminant.

### Separation and quantification step by high performance

### liquid chromatography

Next, the solution treated to remove contaminants in the pretreatment step is analyzed by high performance liquid chromatography (HPLC) to quantify procyanidin and flavan-3-ols contained therein. The column used for the analysis is a reversed-phase column. Examples include, but are not limited to, reversed-phase columns packed with a packing material of a silica gel carrier having octadecyl groups chemically attached thereto (ODS), a silica gel carrier having butyl groups chemically attached thereto (C4), a silica gel carrier having octyl groups chemically attached thereto (C8), a silica gel carrier having phenyl groups chemically attached thereto (Ph), a silica gel carrier having C30 alkyl chains chemically attached thereto (C30), a synthetic polymer carrier having octadecyl groups attached thereto (ODP), a reversed-phase synthetic polymer carrier (e.g., Shodex Rspak DE series (Showa Denko KK, Japan)), and an amide-functionalized reversed-phase carrier (e.g., Ascentis RP-Amide (Sigma-Aldrich Japan KK, Japan)).

The mobile phase used for HPLC analysis may be water and a water-soluble polar solvent. It is possible to use ethanol, methanol, acetonitrile, acetone or the like at a mixing ratio of 0% to 100% with water. The mobile phase is preferably acidified for stabilization of ingredients to be separated and analyzed, and may be mixed with an acid such as trifluoroacetic acid (TFA), formic acid, acetic acid, trichloroacetic acid (TCA) or perchloric acid in an amount of 0.001% to 5%, preferably mixed with 0.05% to 0.1% TFA.

More specifically, an ODS column (Capcellpak C-18 AQ (6 mm × 150 mm, Shiseido Co., Ltd., Japan) is used, and gradient elution is performed at a column temperature of 40°C using 0.05% TFA/water (Eluent A) and 0.05% TFA/90% acetonitrile/water (Eluent B) at a flow rate of 1.2 ml/minute with a gradient of 10% B → 10% B (10 minutes) and then 10% B → 35% B (10 minutes). Detection may be accomplished by measuring the absorbance at 225 nm (A225 nm), so that procyanidin and flavan-3-ols can be quantified from the peak areas of these individual substances. Procyanidin B1 (PB1) (Funakoshi Co., Ltd., Japan) and procyanidin B3 (PB3; synthesized according to Example 4 described later) may be used as standards of procyanidin. The structural formulae of PB1 and PB3 are shown in Figure 1. Under the conditions described above, PB1 is eluted at 10.7 minutes and PB3 at 12.6 minutes. Flavan-3-ols, i.e., (-)-epicatechin, (-)-epicatechin 3-O-gallate, (-)-epigallocatechin, (-)-epigallocatechin 3-O-gallate, (+)-catechin, (-)-catechin 3-O-gallate, (+)-gallocatechin and (-)-gallocatechin 3-O-gallate were purchased from Wako Pure Chemical Industries, Ltd., Japan. (-)-Epicatechin was eluted at 17.6 minutes, (-)-epigallocatechin at 11.1 minutes, (-)-epigallocatechin 3-0-gallate at 18.2 minutes, (+)-catechin at 14.0 minutes, (+)-gallocatechin at 6.4 minutes, and (-)-gallocatechin 3-O-gallate at 19.1 minutes. However, analysis conditions are not limited to those described above, and it is possible to use any conditions which allow quantification.

In another embodiment, the method of the present invention may further comprise mass spectrometer analysis after separation by high performance liquid chromatography. Namely, the pretreated sample may also be quantified by LC-MS. Separation and quantification by LC-MS may be accomplished by using, for example, but not limited to, the conditions shown in Example 6 below. In certain cases such as where only procyanidin dimers are to be quantified by LC-MS, such dimers can be quantified from a chromatogram obtained for the ion at m/z 579([M+H]⁺) by selected ion monitoring (SIM). When the amount of procyanidin contained in a sample is very small (1 ppm or less), LC-MS quantification will be an effective analysis means.

The method of the present invention enables the separation of procyanidin and flavan-3-ols from the other contaminants contained in procyanidin-containing tea products, foods and/or drinks, thereby achieving separation and quantification of procyanidin and flavan-3-ols contained in such tea products and the like. Thus, the method of the present invention is suitable as a method for analyzing procyanidin and flavan-3-ols in tea products, foods and/or drinks, without causing their decomposition.

The present invention will now be described in more detail by way of the following examples, which are not intended to limit the scope of the invention.

### EXAMPLES

### Example 1

### Study of pretreatment conditions

A column containing water-swollen Sephadex LH-20 (dry weight: 0.25 g; Amersham Biosciences) was loaded with 5 ml of an aqueous solution containing proanthocyanidin B1 (PB1) and caffeine (20 ppm each), washed with 2 ml water and then eluted sequentially with 20%, 40%, 60% and 80% EtOH (2 ml each). After concentration under reduced pressure, each eluted fraction was filled up in a 2 ml measuring flask and provided for HPLC (see Example 3 for analysis conditions) to quantify caffeine and PB1.

**Table 1**

| Eluent | % Recovery of caffeine | % Recovery of PB1 |
|---|---|---|
| H₂O (non-adsorbed) | 74% | 0% |
| H₂O (washed) | 23% | 0% |
| 20% EtOH | 2.5% | 0% |
| 40% EtOH | 0% | 0% |
| 60% EtOH | 0% | 49% |
| 80% EtOH | 0% | 27% |

This result indicated that caffeine was substantially eluted with H₂O, whereas PB1 was eluted with 60% or more EtOH. This means that caffeine and PB1 can be separated from each other.

### Example 2

### Pretreatment of tea product on Sephadex LH-20

A column containing water-swollen Sephadex LH-20 (dry weight: 0.25 g; Amersham Biosciences) was loaded with 5 ml of a tea product containing flavangenol (pine bark-derived procyanidin), washed with 2 ml water and then eluted sequentially with 35% EtOH (2 ml) and 70% EtOH (4 ml). The fraction eluted with 70% EtOH was concentrated under reduced pressure and then filled up in a 2 ml measuring flask. Each eluted fraction was provided for HPLC (see Example 3 for analysis conditions) to quantify caffeine and PB1. The concentration was calculated on the basis of the initial volume (5 ml).

**Table 2**

| Eluent | Caffeine concentration | PB1 concentration |
|---|---|---|
| H₂O (non-adsorbed) | 100 ppm | 0 ppm |
| H₂O (washed) | 9 ppm | 0 ppm |
| 35% EtOH | 0.2 ppm | 0.08 ppm |
| 70% EtOH | 0 ppm | 2.7 ppm |

Caffeine was almost completely eluted by washing with water. Subsequently, phenylpropanoids and flavonols were eluted with 35% EtOH, and the desired procyanidin and flavan-3-ol were eluted with 70% EtOH.

It should be noted that it is also possible to use a tea product which has been taken in a volume of 5 ml, freeze-dried and then reconstituted with 5 ml water before use.

### Example 3

### HPLC separation of procyanidin and flavan-3-ols

The fraction treated on Sephadex LH-20 and eluted with water, 35% EtOH or 70% EtOH, as well as the sample solution pretreated to remove contaminants such as caffeine were analyzed by HPLC under the following conditions.

Analysis conditions: Capcellpak C-18 AQ (6 mm × 150 mm, Shiseido Co., Ltd., Japan) was used as a column, and gradient elution was performed using 0.05% TFA/water (Eluent A) and 0.05% TFA/90% CH₃CN/water (Eluent B) at a flow rate of 1.2 ml/minute with a gradient of 10% B → 10% B (10 minutes) and then 10% B → 35% B (10 minutes). The column temperature was set to 40°C, and detection and quantification were based on measurements of A225 nm and its peak area. The HPLC used was Shimadzu LC-2010HT (Shimadzu Corporation, Japan).

Under these conditions, procyanidin B1 was eluted at 10.7 minutes, procyanidin B3 at 12.6 minutes, catechin at 14.0 minutes, epicatechin at 17.6 minutes, gallocatechin at 6.4 minutes, epigallocatechin at 11.1 minutes, epigallocatechin-3-O-gallate at 18.2 minutes, and gallocatechin-3-O-gallate at 19.1 minutes. This result showed good separation of these substances (Figure 2).

### Example 4

### Synthesis of procyanidin B3

Procyanidin B3 was synthesized as follows, according to the article (J. C. S. Perkin I, 1981, Jacobus J. Botha, et al., 1235-1245).

(+)-Taxifolin (500 mg) was dissolved in ethanol (50 ml), followed by addition of NaBH₄ (200 mg). After (+)-catechin (1 g) was added and dissolved, the resulting mixture was mixed with HCl and stirred for 1 hour. The reaction product was purified by reversed-phase HPLC to give procyanidin B3 (200 mg).

### Example 5

### Study of resins for use in pretreatment

A column containing water-equilibrated TOYOPEARL HW-40 (1 ml, Tosoh Corporation, Japan) was loaded with 2 ml of an aqueous solution containing procyanidin B1 (PB1) and caffeine (20 ppm each), washed with 2 ml water and then eluted sequentially with 35% EtOH (2 ml) and 70% EtOH (4 ml). Each eluted fraction was provided for HPLC to quantify caffeine and PB1.

**Table 3**

| Eluent | % Recovery of caffeine | % Recovery of PB1 |
|---|---|---|
| H₂O (Non-adsorbed and washed) | 97.3% | 0% |
| 35% EtOH | 2.5% | 1.3% |
| 70% EtOH | 0% | 77% |

This result indicated that caffeine was substantially eluted with H₂O whereas PB1 was eluted with 70% EtOH, as in the case of Sephadex LH-20. This means that caffeine and PB1 can be separated from each other. This pretreatment method was therefore regarded as an effective method with dextran-based gel filtration resins or hydrophilic vinyl polymer-based resins.

### Example 6

### Quantification with mass spectrometer

The pretreated sample was quantified by LC-MS under the following conditions.

The mass spectrometer used was Quattro micro (Micromass, Manchester, UK) and the measurement was performed in ESI positive mode under the following conditions:

| | |
|---|---|
| Cone voltage | 35 V |
| Collision energy | 2 eV |
| Capillary voltage | 4000 V |
| Source block Temp. | 80°C |
| Desolvation Temp. | 350°C. |

The HPLC used was Aliance 2795 (Nihon Waters KK, Japan). HPLC conditions are as shown below.
Column: Capcell pak C-18 AQ (3 mm φ × 15 cm, Shiseido Co., Ltd., Japan)
Mobile phase: Eluent A: 0.1% HCOOH/water, Eluent B: 90% CH₃CN/0.1% HCOOH/water, flow rate 0.2 ml/minute
Gradient: 10% B → 35% B (7 minutes) → 70% B (3 minutes)

Under these conditions, PB1 was eluted at 7.3 minutes and PB3 at 7.8 minutes. Quantification was performed using a calibration curve prepared for the chromatographic area at m/z 579([M+H]⁺) in selected ion monitoring (SIM). When the amount of procyanidin contained in a sample is very small (1 ppm or less), LC-MS quantification will be an effective analysis means.

## Claims

1. A method for quantifying procyanidin (a generic name for a mixture of catechin n-mer: n ≥ 1) and flavan-3-ols contained in a tea product, foods and/or drinks, which comprises the following steps:
a) pretreating the tea product, foods and/or drinks using a column of a dextran-based or vinyl polymer-based resin to remove contaminants which affect the analysis of procyanidin and flavan-3-ols, wherein said contaminants are selected from methylxanthines, phenylpropanoids, flavones, flavonols, and glycosylated compounds thereof, and wherein the pretreatment step comprises equilibrating the column with water, loading the sample onto the column, eluting contaminants with water and/or 0% to 40% ethanol, and then collecting procyanidin and flavan-3-ols with 60% or more ethanol; and
b) effecting high performance liquid chromatography (HPLC) with a reversed-phase column to separate and quantify procyanidin and flavan-3-ols in the solution treated to remove contaminants during the pretreatment step a).

2. The method according to claim 1, wherein the contaminants are one or more substances selected from the group consisting of caffeine, theobromine, phenylpropanoids, flavonols, and glycosylated compounds of phenylpropanoids and flavonols.

3. The method according to claim 1 or 2, wherein the tea product is a tea beverage containing procyanidin.

4. The method according to claim 1 or 2, wherein the tea product is a tea beverage containing a pine bark extract.

5. The method according to any one of claims 1 to 4, wherein the column used in the pretreatment step is a column of a dextran-based resin.

6. The method according to any one of claims 1 to 5, wherein the reversed-phase column is a column packed with a packing material selected from the group consisting of a silica gel carrier having octadecyl groups chemically attached thereto (ODS), a silica gel carrier having butyl groups chemically attached thereto (C4), a silica gel carrier having octyl groups chemically attached thereto (C8), a silica gel carrier having phenyl groups chemically attached thereto (Ph), a silica gel carrier having C30 alkyl chains chemically attached thereto (C30), a synthetic polymer carrier having octadecyl groups attached thereto (ODP), a reversed-phase synthetic polymer carrier, and an amide-functionalized reversed-phase carrier.

7. The method according to any one of claims 1 to 6, wherein the mobile phase used in step b) for separation by high performance liquid chromatography is acetonitrile and/or water, each of which may or may not contain TFA (trifluoroacetic acid).

8. The method according to claim 7, wherein the mobile phase is a gradient of acetonitrile and water, each of which may or may not contain TFA (trifluoroacetic acid).

9. The method according to any one of claims 1 to 6, wherein the mobile phase used in step b) for separation by high performance liquid chromatography comprises a solvent selected from the group consisting of methanol, ethanol, acetonitrile, acetone and a mixed solvent thereof.

10. The method according to claim 9, wherein the mobile phase used in step b) for separation by high performance liquid chromatography further comprises TFA (trifluoroacetic acid), formic acid, acetic acid, TCA (trichloroacetic acid) or perchloric acid.

11. The method according to any one of claims 1 to 10, wherein procyanidin to be quantified is procyanidin B1 (PB1).

12. The method according to any one of claims 1 to 11, which is further combined with a mass spectrometer in order to quantify only procyanidin dimers.

## Patentansprüche

1. Ein Verfahren zur Quantifizierung von Procyanidin (ein Oberbegriff für ein Gemisch aus Catechin-n-meren: n ≥ 1) und Flavan-3-olen, enthalten in einem Teeprodukt, Lebensmitteln und/oder Getränken, das die folgenden Schritte umfasst:
a) Vorbehandeln des Teeprodukts, der Lebensmittel und/oder Getränke unter Verwendung einer Säule aus einem Harz auf der Basis von Dextran oder auf der Basis eines Vinylpolymers zur Entfernung von Verunreinigungen, welche die Analyse von Procyanidin und Flavan-3-olen beeinflussen, wobei die Verunreinigungen aus Methylxanthinen, Phenylpropanoiden, Flavonen, Flavonolen und glycosylierten Verbindungen davon ausgewählt sind und wobei der Vorbehandlungsschritt Equilibrieren der Säule mit Wasser, Beschicken der Säule mit der Probe, Eluieren von Verunreinigungen mit Wasser und/oder 0 % bis 40 % Ethanol und anschließendes Auffangen von Procyanidin und Flavan-3-olen mit 60 % oder mehr Ethanol umfasst; und
b) Durchführen von Hochleistungsflüssigkeitschromatographie (HPLC) mit einer Umkehrphasensäule zur Trennung und Quantifizierung von Procyanidin und Flavan-3-olen in der Lösung, die während des Vorbehandlungsschrittes a) zur Entfernung von Verunreinigungen behandelt wurde.

2. Das Verfahren gemäß Anspruch 1, wobei die Verunreinigungen eine oder mehrere Substanzen, ausgewählt aus der Gruppe bestehend aus Koffein, Theobromin, Phenylpropanoiden, Flavonolen und glycosylierten Verbindungen von Phenylpropanoiden und Flavonolen, sind.

3. Das Verfahren gemäß Anspruch 1 oder 2, wobei das Teeprodukt ein Teegetränk ist, das Procyanidin enthält.

4. Das Verfahren gemäß Anspruch 1 oder 2, wobei das Teeprodukt ein Teegetränk ist, das einen Kiefernrindenextrakt enthält.

5. Das Verfahren gemäß einem der Ansprüche 1 bis 4, wobei die im Vorbehandlungsschritt verwendete Säule eine Säule aus einem Harz auf der Basis von Dextran ist.

6. Das Verfahren gemäß einem der Ansprüche 1 bis 5, wobei die Umkehrphasensäule eine Säule ist, die mit einem Packmaterial, ausgewählt aus der Gruppe bestehend aus einem Siliciumdioxidgelträger mit chemisch daran gebundenen Octadecylgruppen (ODS), einem Siliciumdioxidgelträger mit chemisch daran gebundenen Butylgruppen (C4), einem Siliciumdioxidgelträger mit chemisch daran gebundenen Octylgruppen (C8), einem Siliciumdioxidgelträger mit chemisch daran gebundenen Phenylgruppen (Ph), einem Siliciumdioxidgelträger mit chemisch daran gebundenen C30-Alkylketten (C30), einem synthetischen Polymerträger mit daran gebundenen Octadecylgruppen (ODP), einem Umkehrphasen-synthetischen Polymerträger und einem Amid-funktionalisierten Umkehrphasenträger, gepackt ist.

7. Das Verfahren gemäß einem der Ansprüche 1 bis 6, wobei die mobile Phase, die in Schritt b) zur Abtrennung durch Hochleistungsflüssigkeitschromatographie verwendet wird, Acetonitril und/oder Wasser ist, von denen jedes TFA (Trifluoressigsäure) enthalten kann oder nicht.

8. Das Verfahren gemäß Anspruch 7, wobei die mobile Phase ein Gradient aus Acetonitril und Wasser ist, von denen jedes TFA (Trifluoressigsäure) enthalten kann oder nicht.

9. Das Verfahren gemäß einem der Ansprüche 1 bis 6, wobei die mobile Phase, die in Schritt b) zur Abtrennung durch Hochleistungsflüssigkeitschromatographie verwendet wird, ein Lösungsmittel, ausgewählt aus der Gruppe bestehend aus Methanol, Ethanol, Acetonitril, Aceton und einem Lösungsmittelgemisch davon, umfasst.

10. Das Verfahren gemäß Anspruch 9, wobei die mobile Phase, die in Schritt b) zur Abtrennung durch Hochleistungsflüssigkeitschromatographie verwendet wird, ferner TFA (Trifluoressigsäure), Ameisensäure, Essigsäure, TCA (Trichloressigsäure) oder Perchlorsäure umfasst.

11. Das Verfahren gemäß einem der Ansprüche 1 bis 10, wobei das zu quantifizierende Procyanidin Procyanidin B 1 (PB 1) ist.

12. Das Verfahren gemäß einem der Ansprüche 1 bis 11, das ferner mit einem Massenspektrometer kombiniert ist, um nur Procyanidindimere zu quantifizieren.

## Revendications

1. Procédé destiné à quantifier la procyanidine (nom générique donné à un mélange de n-mères de catéchine ; n ≥ 1) et les flavan-3-ols contenus dans un produit au thé, des nourritures et/ou des boissons, qui comprend les étapes suivantes :
a) prétraiter le produit au thé, les nourritures et/ou les boissons en utilisant une colonne de résine à base de dextrane ou à base de polymère vinylique afin d'éliminer les contaminants qui affectent l'analyse de la procyanidine et des flavan-3-ols, dans lequel lesdits contaminants sont sélectionnés parmi les méthylxanthines, les phénylpropanoïdes, les flavones, les flavonols, et leurs composés glycosylés, et dans lequel l'étape de prétraitement comprend l'équilibrage de la colonne avec de l'eau, la charge de l'échantillon sur la colonne, l'élution des contaminants avec de l'eau et/ou entre 0 % et 40 % d'éthanol, puis la collecte de la procyanidine et des flavan-3-ols avec 60 % d'éthanol ou plus ; et
b) procéder à une chromatographie liquide à haute performance (HPLC) avec une colonne à phase inverse pour séparer et quantifier la procyanidine et les flavan-3-ols dans la solution traitée afin d'éliminer les contaminants au cours de l'étape de prétraitement a).

2. Procédé selon la revendication 1, dans lequel les contaminants sont une ou plusieurs substances sélectionnées dans le groupe constitué par la caféine, la théobromine, les phénylpropanoïdes, les flavonols, et les composés glycosylés des phénylpropanoïdes et des flavonols.

3. Procédé selon la revendication 1 ou 2, dans lequel le produit au thé est une boisson au thé contenant de la procyanidine.

4. Procédé selon la revendication 1 ou 2, dans lequel le produit au thé est une boisson au thé contenant un extrait d'écorce de pin.

5. Procédé selon l'une quelconque des revendications 1 à 4, dans lequel la colonne utilisée dans l'étape de prétraitement est une colonne de résine à base de dextrane.

6. Procédé selon l'une quelconque des revendications 1 à 5, dans lequel la colonne à phase inverse est une colonne remplie avec un matériau de remplissage sélectionné dans le groupe constitué par un support de gel de silice présentant des groupes octadécyle chimiquement fixés sur celui-ci (ODS), un support de gel de silice présentant des groupes butyle chimiquement fixés sur celui-ci (C4), un support de gel de silice présentant des groupes octyle chimiquement fixés sur celui-ci (C8), un support de gel de silice présentant des groupes phényle chimiquement fixés sur celui-ci (Ph), un support de gel de silice présentant des chaînes alkyle C30 chimiquement fixées sur celui-ci (C30), un support de polymère synthétique présentant des groupes octadécyle fixés sur celui-ci (ODP), un support de polymère synthétique à phase inverse, et un support à phase inverse à fonction amide.

7. Procédé selon l'une quelconque des revendications 1 à 6, dans lequel la phase mobile utilisée dans l'étape b) pour la séparation par la chromatographie liquide à haute performance est de l'acétonitrile et/ou de l'eau, chacun d'eux pouvant contenir ou pas du TFA (acide trifluoroacétique).

8. Procédé selon la revendication 7, dans lequel la phase mobile est un gradient d'acétonitrile et d'eau, chacun d'eux pouvant contenir ou pas du TFA (acide trifluoroacétique).

9. Procédé selon l'une quelconque des revendications 1 à 6, dans lequel la phase mobile utilisée dans l'étape b) pour la séparation par la chromatographie liquide à haute performance, comprend un solvant sélectionné dans le groupe constitué par le méthanol, l'éthanol, l'acétonitrile, l'acétone et un solvant constitué de leur mélange.

10. Procédé selon la revendication 9, dans lequel la phase mobile utilisée dans l'étape b) pour la séparation par la chromatographie liquide à haute performance comprend en outre du TFA (acide trifluoroacétique), de l'acide formique, de l'acide acétique, du TCA (acide trichloracétique) ou de l'acide perchlorique.

11. Procédé selon l'une quelconque des revendications 1 à 10, dans lequel la procyanidine à quantifier est la procyanidine B1 (PB1).

12. Procédé selon l'une quelconque des revendications 1 à 11, qui est en outre combiné à un spectromètre de masse afin de seulement quantifier les dimères de procyanidine.
